# EUROPÄISCHE PATENTANMELDUNG

(11) **EP 3 434 316 A1**
(43) Veröffentlichungstag der Anmeldung: **30.01.2019**
(21) Anmeldenummer: 18181920.2
(22) Anmeldetag: 05.07.2018
(51) Int. Cl.: A61M 39/28, F16K 7/06, F16L 55/10

(54) **SCHLAUCHKLEMME ZUM ABKLEMMEN EINES MEDIZINISCHEN SCHLAUCHES**

(30) Priorität: 26.07.2017 DE 102017212883
(71) Anmelder: B. Braun Melsungen AG, 34212 Melsungen (DE)
(72) Erfinder: FIEGE, Michael, 34121 Kassel (DE); LASCHTOWITZ, Gerrit, 34121 Kassel (DE); TON-THAT, Christine, 34286 Spangenberg (DE)
(74) Vertreter: Patentanwälte Ruff, Wilhelm, Beier, Dauster & Partner mbB

(57) **Zusammenfassung**

1. Schlauchklemme zum Abklemmen eines medizinischen Schlauches
2.1 Eine derartige Schlauchklemme zum Abklemmen eines medizinischen Schlauchs aufweisend zwei Klemmschenkel, die um eine Schwenkachse zwischen einer Öffnungs- und einer Abklemmposition gegeneinander beweglich und in der Abklemmposition mittels eines Rastmittels gegeneinander verrastet sind, einen Durchgangskanal zur Aufnahme des abzuklemmenden Schlauchs, wobei der Durchgangskanal zwischen zwei Durchgangsöffnungen entlang einer Kanallängsrichtung erstreckt und in Bezug auf eine Kanalhochrichtung an einer Ober- und Unterseite jeweils wenigstens abschnittsweise von den Klemmschenkeln bedeckt ist, sowie zwei an der Ober- und Unterseite des Durchgangskanals angeordnete Klemmelemente mit jeweils einer Klemmfläche, die über eine Bewegung der Klemmschenkel in die Abklemmposition derart gegeneinander anstellbar sind, dass der Durchgangskanal in Kanalhochrichtung verengbar und der in dem Durchgangskanal aufnehmbare Schlauch abklemmbar ist, ist bekannt.
2.2 Erfindungsgemäß ist in Bezug auf eine Kanalquerrichtung an gegenüberliegenden Querseiten des Durchgangskanals jeweils ein Begrenzungselement angeordnet, wobei die Begrenzungselemente derart gestaltet sind, dass der Durchgangskanal im Bereich der Klemmflächen in Kanalquerrichtung verschlossen und in Bezug auf die Kanallängsrichtung sowohl vor als auch hinter den Klemmflächen in Kanalquerrichtung wenigstens abschnittsweise freigegeben ist, so dass der abzuklemmende Schlauch in seitlicher Blickrichtung über einen möglichst weiten Bereich des Durchgangskanals von den Begrenzungselementen unverdeckt ist
2.3 Einsatz bei einer Infusionstherapie

## Beschreibung

Die Erfindung betrifft eine Schlauchklemme zum Abklemmen eines medizinischen Schlauches aufweisend zwei Klemmschenkel, die um eine Schwenkachse zwischen einer Öffnungs- und einer Abklemmposition gegeneinander beweglich und in der Abklemmposition mittels eines Rastmittels gegeneinander verrastet sind, einen Durchgangskanal zur Aufnahme des abzuklemmenden Schlauches, wobei der Durchgangskanal zwischen zwei Durchgangsöffnungen entlang einer Kanallängsrichtung erstreckt und in Bezug auf eine Kanalhochrichtung an einer Ober- und Unterseite jeweils wenigstens abschnittsweise von den Klemmschenkeln bedeckt ist, sowie zwei an der Ober- und Unterseite des Durchgangskanals angeordnete Klemmelemente mit jeweils einer Klemmfläche, die über eine Bewegung der Klemmschenkel in die Abklemmposition derart gegeneinander anstellbar sind, dass der Durchgangskanal in Kanalhochrichtung verengbar und der in dem Durchgangskanal aufnehmbare Schlauch abklemmbar ist.

Eine derartige Schlauchklemme ist aus der EP 0 517 000 A2 bekannt. Die bekannte Schlauchklemme weist einen bügelförmigen Grundkörper mit einem oberen und einem unteren Klemmschenkel jeweils in Form eines Bügelschenkels sowie zwei stirnendseitige Durchgangsöffnungen auf. In Hochrichtung zwischen den Bügelschenkeln erstreckt sich ein Durchgangskanal der zur Aufnahme eines abzuklemmenden medizinischen Schlauches vorgesehen ist. An den beiden Bügelschenkeln ist jeweils innenliegend ein Klemmelement in Form einer keilförmigen Klemmbacke mit jeweils einer im Wesentlichen wulstartigen Klemmfläche angeformt. Zum Abklemmen des Schlauches werden die Bügelschenkel auf bekannte Weise mittels Fingerdruck gegeneinander bewegt und mittels eines Rastmittels aufweisend eine Rastzunge und eine Raststufe gegeneinander verrastet. Derart wird der zwischen den Klemmflächen liegende Abschnitt des Durchgangskanals in Kanalhochrichtung verengt, so dass ein in dem Durchgangskanal angeordneter Schlauch abklemmbar ist. Zusätzlich zu dem Grundkörper ist ein separater Einsatzkörper vorgesehen, der zwischen den Bügelschenkeln einklipsbar ist. Dieser Einsatzkörper soll verhindern, dass der abzuklemmende Schlauch in Querrichtung über die Klemmflächen hinaus ausgewölbt und infolgedessen nicht oder nur unzureichend geklemmt wird.

Aufgabe der Erfindung ist es, eine Schlauchklemme der eingangs genannten Art zu schaffen, die verbesserte Eigenschaften aufweist.

Diese Aufgabe wird dadurch gelöst, dass in Bezug auf eine Kanalquerrichtung an gegenüberliegenden Querseiten des Durchgangskanals jeweils ein Begrenzungselement angeordnet ist, wobei die Begrenzungselemente derart gestaltet sind, dass der Durchgangskanal im Bereich der Klemmflächen in Kanalquerrichtung verschlossen und in Bezug auf die Kanallängsrichtung sowohl vor als auch hinter den Klemmflächen in Kanalquerrichtung wenigstens abschnittsweise freigegeben ist, so dass der abzuklemmende Schlauch in seitlicher Blickrichtung über einen möglichst weiten Bereich des Durchgangskanals von den Begrenzungselementen unverdeckt ist. Durch die erfindungsgemäße Lösung wird zum einen der Durchgangskanal im Bereich der Klemmflächen in Kanalquerrichtung verschlossen. Dies verhindert, dass sich der abzuklemmende Schlauch seitlich zwischen den Klemmflächen auswölben kann und infolgedessen nicht oder nur teilweise geklemmt wird. Auf diese Weise wird eine funktionssichere Klemmung gewährleistet. Zum anderen wird durch die erfindungsgemäße Lösung erreicht, dass der Durchgangskanal in Bezug auf die Kanallängsrichtung vor und hinter den Klemmflächen in Kanalquerrichtung freigegeben ist. Mit anderen Worten: Der abzuklemmende Schlauch bleibt in seitlicher Blickrichtung über einen möglichst weiten Bereich des Durchgangskanals von den Begrenzungselementen unverdeckt. Auf diese Weise bleibt der abzuklemmende Schlauch trotz des erfindungsgemäßen Verschlusses in Querrichtung mittels der Begrenzungselemente von außen gut einsehbar. Medizinisches Personal kann demzufolge auf einfache Weise überprüfen, ob eine ordnungsgemäße Klemmung des Schlauches erreicht ist oder nicht. Die Klemmelemente können jeweils in Gestalt keilförmiger oder abgerundeter Klemmbacken gestaltet sein. Die Klemmflächen können vorteilhafterweise konvex, wulstförmig oder kreiszylinderflächenartig gestaltet sein. Das Rastmittel kann vorteilhafterweise eine Rastnase und einen Rasthaken umfassen, die in der Abklemmposition der Schlauchklemme gegeneinander verrastet sind. Sowohl die Rastnase als auch der Rasthaken können jeweils stirnendseitig an jeweils einem der Klemmschenkel angeordnet, insbesondere einstückig angeformt, sein. Die Begrenzungselemente können jeweils an einem in Bezug auf die Kanalhochrichtung oberen oder unteren der Klemmschenkel angeordnet sein. Alternativ kann an beiden der Klemmschenkel jeweils eines der Begrenzungselemente angeordnet sein.

In Ausgestaltung der Erfindung sind die Begrenzungselemente in Kanallängsrichtung jeweils in etwa über die Länge der Klemmelemente, vorzugsweise über die Länge der Klemmflächen, erstreckt. Bei dieser Ausgestaltung der Erfindung findet nach wie vor eine hinreichende Stützung des abzuklemmenden Schlauchs in Kanalquerrichtung statt, so dass dieser an einem seitlichen Auswölben zwischen den Klemmflächen gehindert wird. Andererseits wird durch die derartige Bemessung der Länge der Begrenzungselemente eine besonders gute Einsehbarkeit des abzuklemmenden Schlauchs in seitlicher Blickrichtung erreicht.

In weiterer Ausgestaltung der Erfindung sind die Begrenzungselemente in Kanallängsrichtung jeweils über maximal die doppelte Länge, vorzugsweise über maximal die 1,5-fache Länge, der

Klemmflächen erstreckt. Diese Ausgestaltung der Erfindung erreicht eine nochmals verbesserte Einsehbarkeit des abzuklemmenden Schlauchs in seitlicher Blickrichtung. Zudem kann durch eine derart längenmäßig begrenzte Ausgestaltung der Begrenzungselemente Material eingespart und somit eine kostengünstige Fertigung erreicht werden.

In weiterer Ausgestaltung der Erfindung sind die Begrenzungselemente jeweils in Form einer im Wesentlichen in Kanalhochrichtung orientierten Leiste ausgebildet. Vorteilhafterweise können die derart leistenförmig ausgebildeten Begrenzungselemente an ihren in Bezug auf die Kanalhochrichtung jeweils unteren Stirnendbereichen eine Abrundung aufweisen. Sofern ein abzuklemmender Schlauch unbeabsichtigt zwischen eines der Begrenzungselemente und eines der Klemmelemente gelangt, kann infolge der Abrundung eine Beschädigung des Schlauchs vermieden werden.

In weiterer Ausgestaltung der Erfindung weisen die Begrenzungselemente jeweils eine Vorder- und eine Rückkante auf, die zueinander parallel oder keilförmig verlaufen. Demzufolge wird eine besonders schlanke Gestaltung der Begrenzungselemente erreicht, wodurch die seitliche Einsehbarkeit des abzuklemmenden Schlauchs nochmals verbessert wird.

In weiterer Ausgestaltung der Erfindung sind die Begrenzungselemente in Kanalquerrichtung jeweils flächenbündig mit oder zurückversetzt zu jeweils einem außenliegenden Wandabschnitt eines der Klemmschenkel. Durch diese Ausgestaltung der Erfindung wird eine Schlauchklemme mit beidseits glattflächigen Stirnflächen bzw. ohne seitliche Abragungen erreicht. Für Anwendungsfälle, bei denen die Schlauchklemme in Kontakt mit der Haut eines Patienten steht, wird demzufolge ein verbesserter Komfort erreicht und Druckstellen werden vermieden.

In weiterer Ausgestaltung der Erfindung sind die Begrenzungselemente an dem in Bezug auf die Kanalhochrichtung oberen der Klemmschenkel angeordnet. Sofern der obere Klemmschenkel stirnendseitig eine Rastnase des Rastmittels aufweist, kann durch eine derartige Anordnung der Begrenzungselemente unter Umständen eine verbesserte Fertigbarkeit der Schlauchklemme erreicht werden.

In weiterer Ausgestaltung der Erfindung sind die Begrenzungselemente alternierend an dem oberen oder dem unteren der Klemmschenkel angeordnet. Diese Ausgestaltung sieht das eine Begrenzungselement an dem oberen Klemmschenkel und das andere Begrenzungselement versetzt gegenüberliegend an dem unteren Klemmschenkel vor. Diese Ausgestaltung ermöglicht eine vereinfachte Herstellbarkeit.

In weiterer Ausgestaltung der Erfindung sind die Begrenzungselemente einstückig mit den Klemmschenkeln und den Klemmelementen ausgebildet. Insoweit ist es besonders vorteilhaft, wenn die Schlauchklemme als einstückiges Bauteil ausgebildet ist, insbesondere als Kunststoffspritzgussbauteil. Diese Ausgestaltung erlaubt zum einen eine besonders kostengünstige Fertigung. Zum anderen wird durch die einstückige Anformung der Begrenzungselemente an die weiteren Bau- und/oder Formelemente der Schlauchklemme eine kompakte Bauweise erreicht. Zudem wird derart einem unbeabsichtigten Ablösen der Begrenzungselemente von der Schlauchklemme vorgebeugt, was beispielsweise bei einer Ausgestaltung der Begrenzungselemente in Form eines Einsatzkörpers nicht unter allen Umständen ausgeschlossen werden kann.

In weiterer Ausgestaltung der Erfindung entspricht die Erstreckung der Begrenzungselemente in Kanalhochrichtung wenigstens der Höhe des Durchgangskanals zwischen den Klemmflächen, wenn die Klemmschenkel in der Öffnungsposition sind. Mit anderen Worten: Die Begrenzungselemente sind derart gestaltet, dass der Durchgangskanal im Bereich der Klemmflächen in Kanalquerrichtung auch dann verschlossen ist, wenn die Klemmschenkel die Öffnungsposition einnehmen. Dies ist eine besonders funktionssichere Ausgestaltung der Erfindung, da derart einem seitlichen Auswölben des abzuklemmenden Schlauchs auch in der Öffnungsposition der Klemmschenkel entgegengewirkt wird.

In weiterer Ausgestaltung der Erfindung weisen die Begrenzungselemente jeweils einen Wandabschnitt auf, der gegen jeweils einen Gleitwandabschnitt eines der Klemmschenkel und/oder der Klemmelemente in Kanalquerrichtung abgestützt und senkrecht zu dieser gleitbeweglich ist. Die Wandabschnitte der Begrenzungselemente können vorteilhafterweise jeweils ein dem Durchgangskanal zugewandter Wandabschnitt sein. Dementsprechend kann der Gleitwandabschnitt vorteilhafterweise ein dem Durchgangskanal abgewandter Wandabschnitt eines der Klemmschenkel und/oder eines der Klemmelemente sein. Durch die derartige Abstützung der Begrenzungselemente in Querrichtung wird eine verbesserte Führung der Klemmschenkel bei einer Bewegung zwischen der Öffnungs- und der Abklemmposition erreicht. Auf diese Weise kann einem ungewollten Verkanten der Schlauchklemme bei der Betätigung entgegengewirkt werden.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1 bis 3: zeigen in schematischer Perspektivdarstellung (Fig. 1), einer Seitenansicht (Fig. 2) sowie einer Schnittdarstellung (Fig. 3) in einer Schnittebene B-B gemäß Fig. 2 eine Ausführungsform einer erfindungsgemäßen Schlauchklemme in einer Öffnungsposition,
- Fig. 4 bis 6: die Schlauchklemme nach den Fig. 1 bis 3 in einer Abklemmposition in einer schematischen Perspektivdarstellung (Fig. 4), einer Seitenansicht (Fig. 5) sowie einer Schnittdarstellung (Fig. 6) entlang einer Schnittebene A-A gemäß Fig. 5 und
- Fig. 7 bis 9: eine weitere Ausführungsform einer erfindungsgemäßen Schlauchklemme ähnlich den Fig. 4 bis 6.

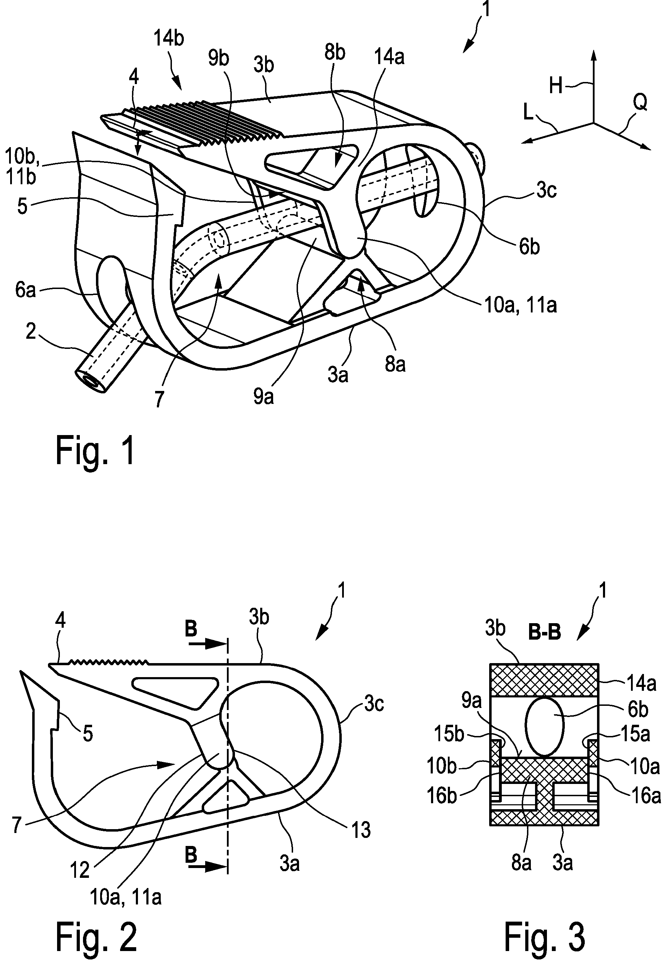

Eine Schlauchklemme 1 nach den Fig. 1 bis 6 ist zum Abklemmen eines medizinischen Schlauchs 2 (Fig. 1) insbesondere bei einer Infusionstherapie vorgesehen.

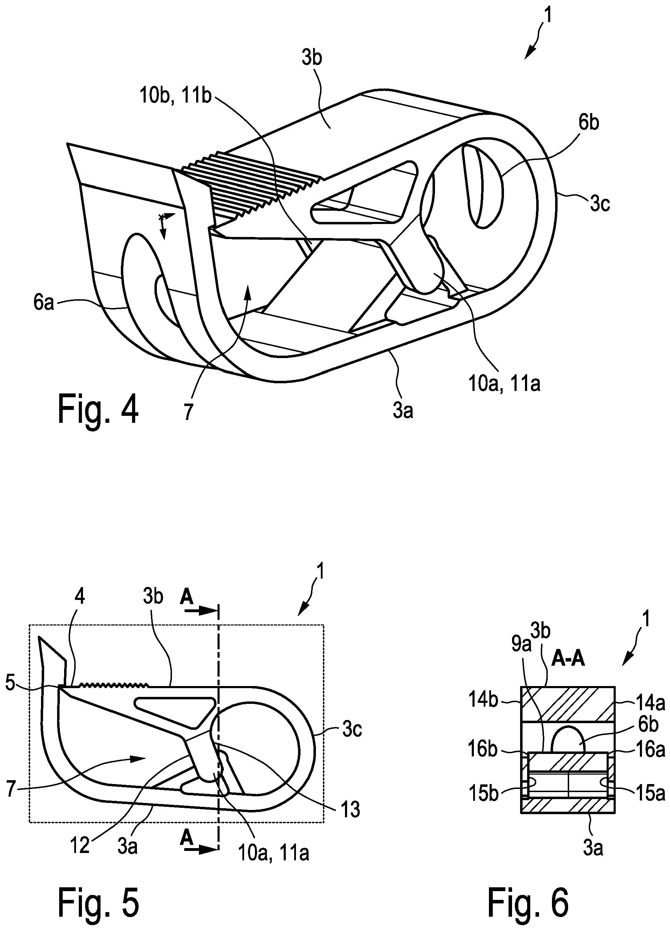

Die Schlauchklemme 1 weist zwei Klemmschenkel 3a, 3b auf. Die beiden Klemmschenkel 3a, 3b sind über einen bogenförmigen, flexiblen Wandabschnitt 3c miteinander verbunden und insoweit um eine nicht näher bezeichnete Schwenkachse zwischen einer Öffnungsposition (Fig. 1 bis 3) und einer Abklemmposition (Fig. 4 bis 6) gegeneinander beweglich. Der in Bezug auf die Zeichenebene der Fig. 1 bis 6 obere Klemmschenkel 3b weist stirnendseitig eine Rastnase 4 auf. Dementsprechend weist der untere Klemmschenkel 3a stirnendseitig einen Rasthaken 5 auf. Die Rastnase 4 und der Rasthaken 5 bilden ein Rastmittel 4, 5 der Schlauchklemme 1, über das die Klemmschenkel 3a, 3b in der Abklemmposition gegeneinander verrastet sind. Weiter weist die Schlauchklemme 1 eine vordere Durchgangsöffnung 6a sowie eine hintere Durchgangsöffnung 6b auf. Die Durchgangsöffnungen 6a, 6b sind jeweils in Form eines im Wesentlichen elliptischen Durchlasses ausgebildet und in der Wandung des unteren Klemmschenkels 3a bzw. der Wandung des oberen Klemmschenkels 3b und des bogenförmigen Wandabschnitts 3c angeordnet. Zumindest abschnittsweise entlang einer Kanallängsrichtung L ist ein Durchgangskanal 7 zur Aufnahme des abzuklemmenden Schlauchs 2 zwischen den Durchgangsöffnungen 6a, 6b erstreckt. In Bezug auf eine Kanalhochrichtung H ist der Durchgangskanal 7 an einer Ober- und Unterseite jeweils wenigstens abschnittsweise von den Klemmschenkeln 3a, 3b bedeckt. Zudem weist die Schlauchklemme 1 an den dem Durchgangskanal 7 zugewandten Wandabschnitten der Klemmschenkel 3a, 3b angeordnete Klemmelemente 8a, 8b auf. Die Klemmelemente 8a, 8b sind in Bezug auf die Kanalhochrichtung H an gegenüberliegenden Seiten des Durchgangskanals 7 angeordnet und weisen jeweils eine Klemmfläche 9a, 9b auf. Die Klemmelemente 8a, 8b sind jeweils in Form eines keilförmig gestalteten Klemmbackens ausgebildet. Die Klemmflächen 9a, 9b weisen jeweils die Form eines Zylindermantelabschnitts auf und sind insoweit konvex oder wulstförmig gestaltet. Über eine Bewegung der Klemmschenkel 3a, 3b in die Abklemmposition, wie sie in bekannter Weise durch Fingerdruck auf die Außenflächen der Klemmschenkel 3a, 3b bewirkbar ist, sind die Klemmflächen 9a, 9b gegeneinander anstellbar. Infolge dieser Anstellung ist der Durchgangskanal 7 in Kanalhochrichtung H im Bereich der Klemmfläche 9a, 9b verengbar und somit der abzuklemmende Schlauch 2 an dem zwischen den Klemmflächen 9a, 9b angeordneten Schlauchmantelabschnitt abklemmbar. Auf diese Weise bildet die Schlauchklemme 1 eine Art Absperrglied mit zwei Funktionsstellungen (geöffnet, geschlossen) für einen durch den Schlauch 2 strömenden Volumenstrom.

Weiter weist die Schlauchklemme 1 zwei Begrenzungselemente 10a, 10b auf. Die Begrenzungselemente 10a, 10b sind in Bezug auf eine Kanalquerrichtung Q an gegenüberliegenden Querseiten der Schlauchklemme 1 und insoweit an gegenüberliegenden Querseiten des Durchgangskanals 7 angeordnet. Die Begrenzungselemente 10a, 10b sind derart gestaltet, dass der Durchgangskanal 7 im Bereich der Klemmflächen 9a, 9b in Kanalquerrichtung Q verschlossen ist. Auf diese Weise wird einem ungewollten seitlichen Auswölben des abzuklemmenden Schlauchs 2 und somit einem Bypass oder einem lediglich teilweisen Abklemmen des Schlauchs 2 entgegengewirkt. Zudem sind die Begrenzungselemente 10a, 10b derart gestaltet, dass der Durchgangskanal 7 in Bezug auf die Kanallängsrichtung L sowohl vor als auch hinter den Klemmflächen 9a, 9b in Kanalquerrichtung Q wenigstens abschnittsweise freigegeben ist. Dies gewährleistet eine leichte Einsehbarkeit des Klemmbereichs zwischen den Klemmflächen 9a, 9b, so dass medizinisches Personal eine funktionsgerechte Klemmung des Schlauchs 2 jederzeit ohne weiteres überprüfen kann.

Die Begrenzungselemente 10a, 10b sind einstückig mit den Klemmschenkeln 3a sowie 3b und den Klemmelementen 8a, 8b ausgebildet, so dass eine einteilige Ausführung der Schlauchklemme 1 erreicht ist. Die Schlauchklemme 1 kann vorzugsweise als Kunststoffspritzgussbauteil hergestellt sein.

Weiter sind die Begrenzungselemente 10a, 10b jeweils in Form einer Leiste 11a, 11b ausgebildet. Diese Leisten 11a, 11b sind jeweils als eine Art Fortsatz an den gegenüberliegenden Seiten des Klemmelements 8b und somit an dem oberen Klemmschenkel 3b angeordnet.

In Bezug auf die Kanallängsrichtung L sind die Begrenzungselemente 10a, 10b jeweils in etwa über die Länge der Klemmflächen 9a, 9b erstreckt. Sofern die Klemmelemente 8a, 8b anstelle der keilförmigen Ausgestaltung als vertikale Wandabschnitte ausgebildet sind, kann es ausreichen, wenn die Begrenzungselemente 10a, 10b in Kanallängsrichtung L jeweils in etwa über die Länge der Klemmelemente 8a, 8b erstreckt sind. Vorliegend beträgt die jeweilige Länge der Begrenzungselemente 10a, 10b jedenfalls nicht mehr als die 1,5-fache Länge der Klemmflächen 9a, 9b. Derart wird eine besonders gute Einsehbarkeit des abzuklemmenden Schlauchs im Bereich der Klemmflächen 9a, 9b erreicht.

Die Begrenzungselemente 10a, 10b weisen jeweils eine Vorderkante 12 und eine Rückkante 13 auf. Die Vorderkante 12 und die Rückkante 13 verlaufen im Wesentlichen parallel zueinander, wobei selbstverständlich auch hiervon abweichende Kantenverläufe möglich sind. Besonders bevorzugt können die Vorderkante 12 und die Rückkante 13 keilförmig zueinander verlaufen, wobei sich der Abstand zwischen der Vorderkante 12 und der Rückkante 13 in Bezug auf die Kanalhochrichtung H von oben nach unten verjüngt.

Die Begrenzungselemente 10a, 10b sind in Kanalquerrichtung Q jeweils flächenbündig zu einem außenliegenden Wandabschnitt 14a bzw. 14b des oberen Klemmschenkels 3b. Weiter weisen die Begrenzungselemente 10a, 10b jeweils einen innenliegenden Wandabschnitt 15a bzw. 15b auf. Diese Wandabschnitte 15a, 15b sind in Bezug auf die Kanalquerrichtung Q jeweils gegen einen Gleitwandabschnitt 16a bzw. 16b abgestützt. Die beiden Gleitwandabschnitte 16a, 16b sind außenliegende Stirnflächen des an dem unteren Klemmschenkel 3a angeordneten Klemmelements 8a. Bei einer Bewegung der Klemmschenkel 3a, 3b zwischen der Öffnungs- und der Abklemmposition sind die Begrenzungselemente 10a, 10b über ihre innenliegenden Wandabschnitte 15a, 15b gleitbeweglich gegen die Gleitwandabschnitte 16a, 16b abgestützt. Auf diese Weise wird eine seitliche Führung der Klemmschenkel 3a, 3b erreicht und einem Verkanten der Schlauchklemme 1 entgegengewirkt.

Wie weiter insbesondere anhand der Fig. 1 bis 3 ersichtlich ist, entspricht die Erstreckung der Begrenzungselemente 10a, 10b in Kanalhochrichtung H wenigstens der Höhe des Durchgangskanals 7 zwischen den Klemmflächen 9a, 9b, wenn die Klemmschenkel 3a, 3b in der Öffnungsposition sind. Mit anderen Worten: Die Begrenzungselemente 10a, 10b sind derart gestaltet, dass der Durchgangskanal 7 im Bereich der Klemmflächen 9a, 9b in Kanalquerrichtung Q stets und insoweit unabhängig von der Position der Klemmschenkel 3a, 3b mittels der Begrenzungselemente 10a, 10b verschlossen ist.

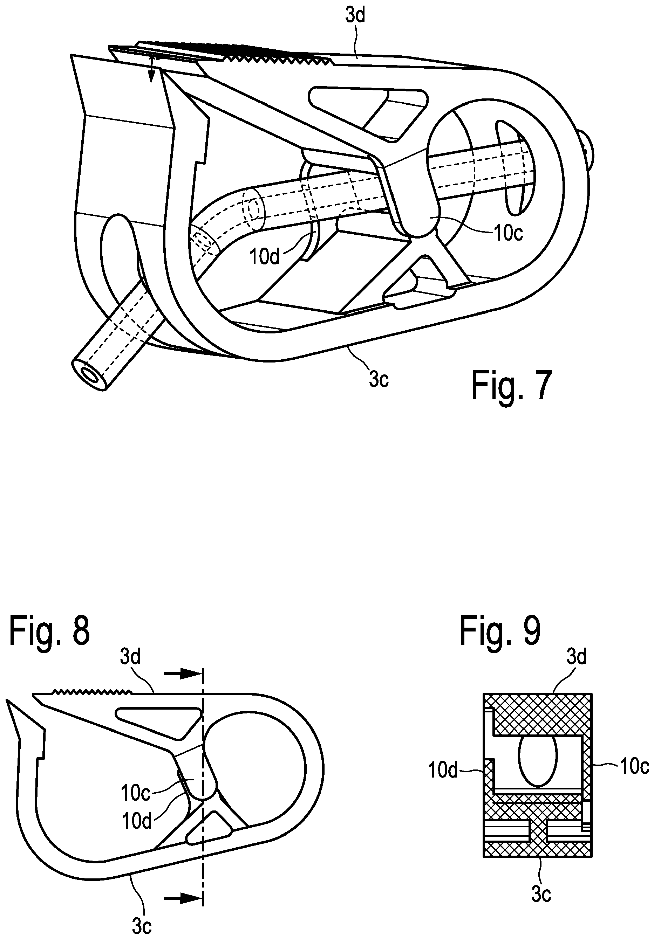

Die Schlauchklemme gemäß den Fig. 7 bis 9 entspricht weitgehend der Schlauchklemme 1 gemäß den Fig. 1 bis 6. Zur Vermeidung von Wiederholungen wird daher ergänzend auf die Beschreibung zu der Schlauchklemme gemäß den Fig. 1 bis 6 verwiesen. Nachfolgend wird auf die Unterschiede der Schlauchklemme gemäß den Fig. 7 bis 9 eingegangen, wobei Fig. 9 eine Schnittdarstellung entlang der Schnittlinie in Fig. 8 zeigt.

Die Schlauchklemme gemäß den Fig. 7 bis 9 weist einen oberen Klemmschenkel 3d und einen unteren Klemmschenkel 3c auf. Jedem Klemmschenkel 3c, 3d ist seitlich außen jeweils ein Begrenzungselement 10c, 10d zugeordnet, die einander diametral gegenüber liegen und in Querrichtung versetzt jeweils zu dem anderen Klemmschenkel hin abragen. Die Begrenzungselemente 10c, 10d sind demzufolge alternierend an dem oberen Klemmschenkel 3d oder dem unteren Klemmschenkel 3c angeordnet. Dies bedeutet, dass das Begrenzungselement 10c gemäß der Darstellung nach den Fig. 7 bis 9 einstückig an dem oberen Klemmschenkel 3d angeformt ist, während das andere Begrenzungselement 10d an dem unteren Klemmschenkel 3c angeformt ist. Die Ausrichtung der Begrenzungselemente 10c, 10d und die entsprechende Überlappung im Bereich von Klemmflächen entspricht der Ausführungsform nach den Fig. 1 bis 6.

## Patentansprüche

1. Schlauchklemme (1) zum Abklemmen eines medizinischen Schlauches (2) aufweisend zwei Klemmschenkel (3a, 3b), die um eine Schwenkachse zwischen einer Öffnungs- und einer Abklemmposition gegeneinander beweglich und in der Abklemmposition mittels eines Rastmittels (4, 5) gegeneinander verrastet sind, einen Durchgangskanal (7) zur Aufnahme des abzuklemmenden Schlauches (2), wobei der Durchgangskanal (7) zwischen zwei Durchgangsöffnungen (6a, 6b) entlang einer Kanallängsrichtung (L) erstreckt und in Bezug auf eine Kanalhochrichtung (H) an einer Ober- und Unterseite jeweils wenigstens abschnittsweise von den Klemmschenkeln (3a, 3b) bedeckt ist, sowie zwei an der Ober- und Unterseite des Durchgangskanals (7) angeordnete Klemmelemente (8a, 8b) mit jeweils einer Klemmfläche (9a, 9b), die über eine Bewegung der Klemmschenkel (3a, ,3b) in die Abklemmposition derart gegeneinander anstellbar sind, dass der Durchgangskanal (7) in Kanalhochrichtung (H) verengbar und der in dem Durchgangskanal (7) aufnehmbare Schlauch (2) abklemmbar ist, **dadurch gekennzeichnet, dass** in Bezug auf eine Kanalquerrichtung (Q) an gegenüberliegenden Querseiten des Durchgangskanals (7) jeweils ein Begrenzungselement (10a, 10b) angeordnet ist, wobei die Begrenzungselemente (10a, 10b) derart gestaltet sind, dass der Durchgangskanal (7) im Bereich der Klemmflächen (9a, 9b) in Kanalquerrichtung (Q) verschlossen und in Bezug auf die Kanallängsrichtung (L) sowohl vor als auch hinter den Klemmflächen (9a, 9b) in Kanalquerrichtung (Q) wenigstens abschnittsweise freigegeben ist, so dass der abzuklemmende Schlauch (2) in seitlicher Blickrichtung über einen möglichst weiten Bereich des Durchgangskanals (7) von den Begrenzungselementen (10a, 10b) unverdeckt ist.

2. Schlauchklemme (1) nach Anspruch 1, **dadurch gekennzeichnet, dass** die Begrenzungselemente (10a, 10b) in Kanallängsrichtung (L) jeweils in etwa über die Länge der Klemmelemente (8a, 8b), vorzugsweise über die Länge der Klemmflächen (9a, 9b), erstreckt sind.

3. Schlauchklemme (1) nach Anspruch 1 oder 2, **dadurch gekennzeichnet, dass** die Begrenzungselemente (10a, 10b) in Kanallängsrichtung (L) jeweils über maximal die doppelte Länge, vorzugsweise über maximal die 1,5-fache Länge, der Klemmflächen (9a, 9b) erstreckt sind.

4. Schlauchklemme (1) nach einem der vorhergehenden Ansprüche, **dadurch gekennzeichnet, dass** die Begrenzungselemente (10a, 10b) jeweils in Form einer im Wesentlichen in Kanalhochrichtung orientierten Leiste (11a, 11b) ausgebildet sind.

5. Schlauchklemme (1) nach einem der vorhergehenden Ansprüche, **dadurch gekennzeichnet, dass** die Begrenzungselemente (10a, 10b) jeweils eine Vorder- (12) und eine Rückkante (13) aufweisen, die zueinander parallel oder keilförmig verlaufen.

6. Schlauchklemme (1) nach einem der vorhergehenden Ansprüche, **dadurch gekennzeichnet, dass** die Begrenzungselemente (10a, 10b) in Kanalquerrichtung (Q) jeweils flächenbündig mit oder zurückversetzt sind zu jeweils einem außenliegenden Wandabschnitt (14a, 14b) eines der Klemmschenkel (3a, 3b).

7. Schlauchklemme (1) nach einem der vorhergehenden Ansprüche, **dadurch gekennzeichnet, dass** die Begrenzungselemente (10a, 10b) an dem in Kanalhochrichtung (H) oberen der Klemmschenkel (3b) angeordnet sind.

8. Schlauchklemme (1) nach einem der vorhergehenden Ansprüche, **dadurch gekennzeichnet, dass** die Begrenzungselemente (10c, 10d) alternierend an dem oberen oder dem unteren der Klemmschenkel (3c, 3d) angeordnet sind.

9. Schlauchklemme (1) nach einem der vorhergehenden Ansprüche, **dadurch gekennzeichnet, dass** die Begrenzungselemente (10a, 10b) einstückig mit den Klemmschenkeln (3a, 3b) und den Klemmelementen (8a, 8b) ausgebildet sind.

10. Schlauchklemme (1) nach einem der vorhergehenden Ansprüche, **dadurch gekennzeichnet, dass** die Erstreckung der Begrenzungselemente (10a, 10b) in Kanalhochrichtung (H) wenigstens der Höhe des Durchgangskanals (7) zwischen den Klemmflächen (9a, 9b) entspricht, wenn die Klemmschenkel (3a, 3b) in der Öffnungsposition sind.

11. Schlauchklemme (1) nach einem der vorhergehenden Ansprüche, **dadurch gekennzeichnet, dass** die Begrenzungselemente (10a, 10b) jeweils einen Wandabschnitt (15a, 15b) aufweisen, der gegen jeweils einen Gleitwandabschnitt (16a, 16b) eines der Klemmschenkel (3a, 3b) und/oder der Klemmelemente (8a, 8b) in Kanalquerrichtung (Q) abgestützt und senkrecht zu dieser gleitbeweglich ist.
